# EUROPEAN PATENT SPECIFICATION

(11) **EP 3 389 751 B1**
(45) Date of publication and mention of the grant of the patent: **11.12.2024**
(21) Application number: 17713466.5
(22) Date of filing: 22.03.2017
(51) Int. Cl.: A61F 9/00, A61M 3/02, A61M 5/36, A61M 5/38

(54) **FLUID DELIVERY SYSTEM**
FLÜSSIGKEITSABGABESYSTEM
SYSTEME D'ADMINISTRATION DE FLUIDE

(30) Priority: 22.03.2016 US 201615077575
(43) Date of publication of application: 24.10.2018
(73) Proprietor: Alcon Inc., 1701 Fribourg (CH)
(72) Inventor: SANCHEZ, JR., Robert Joseph, Carlsbad, California 92010 (US)
(74) Representative: Murgitroyd & Company
(86) International application number: PCT/IB2017/051664
(87) International publication number: WO 2017/163202

(56) References cited:
- WO-A1-2013/151904
- US-A1- 2010 030 151
- US-A1- 2010 331 779

## Description

### TECHNICAL FIELD

The present disclosure is directed to systems for performing ophthalmic surgical procedures, and more particularly, to systems for providing pressurized fluid for infusion into a patient's eye.

### BACKGROUND

Fluids are typically injected into a patient's eye during an ophthalmic surgery in order to maintain the intraocular pressure of the eye at an acceptable level. Some ophthalmic surgical systems provide such fluid from a bag placed under physical pressure by an actuator mechanism. The actuator mechanism squeezes the bag to push fluid out of the bag and into an infusion line. The infusion line provides fluid communication between the bag and the ophthalmic surgical tool that injects the fluid into the patient's eye. Some ophthalmic surgical systems provide such fluid through use of a bottle. Typically, the bottle has an infusion outflow port at the bottom of the bottle, which can be connected to the fluid infusion line. The bottle also has a pressure inlet at the top of the bottle. The pressure inlet is connected to a pressurized fluid source such as a pressurized gas. When the pressurized gas is injected into the bottle, it pushes fluid out of the infusion outflow port. In some examples, the fluid is pushed into a cassette chamber. In some examples, the fluid is pushed into the fluid infusion line.

The ophthalmic surgical systems that provide fluid infusion, among other functions, typically do so through use of a fluid delivery system integrated with a console. For example, the fluid delivery system for an ophthalmic surgical system may include a space for placing the bag or bottle while the bag or bottle is connected to the fluid delivery system. In some cases, the bags or bottles may come packaged with the infusion fluid therein. However, such fluid is not typically degassed. In other words, there may be gas bubbles within the infusion fluid or gas that is dissolved into the infusion fluid. An infusion fluid that is not degassed may introduce gas bubbles into the patient's eye during the infusion process. Such gas bubbles may obscure an operator's vision during the ophthalmic surgical operation being performed.

Reference is made to US20100331779 and WO2013/151904 which have been cited as relating to the state of the art. US20100331779 discloses a medical fluid injection system, comprising: two medical fluid reservoirs and two pressurizing units.

### SUMMARY

It will be appreciated that the scope of the invention is in accordance with the claims. Accordingly, there is provided a fluid delivery system, as defined in claim 1. Further there is provided a method as defined in claim 4. Further features are provided in the dependent claims.

According to one example, a fluid delivery system includes a pressure source capable of producing both positive and negative fluid pressure, a fluid infusion line, and a first fluid storage container. The first fluid storage container includes a chamber, a fluid outflow port that is connectable to the fluid infusion line to provide fluid communication between the chamber and the fluid infusion line, a pressure inlet that is connectable to the pressure source, and a filter disposed between the pressure inlet and the chamber. The fluid delivery system further includes a control system configured to cause the fluid pressure source to apply both negative pressure and positive pressure.

A method includes connecting a pressure source of a fluid delivery system to a pressure inlet of a fluid storage container, the pressure inlet comprising a filter that allows gas to pass therethrough, connecting a fluid infusion line of the fluid delivery system to a fluid outflow port of the fluid storage container, and applying a negative pressure to the fluid storage container to degas a liquid within the fluid storage container.

A fluid delivery system includes a pressure source capable of producing both positive and negative fluid pressure, a fluid infusion line, a fluid source, and a first fluid storage container. The first fluid storage container includes a first fluid storage chamber, a first infusion outflow port that is connectable to the fluid infusion line, a first fluid inflow port that is connectable to the fluid source, and a first pressure inlet that is connectable to the fluid pressure source, the first pressure inlet comprising a first filter. The fluid delivery system further includes a control system configured to fill the fluid storage chamber with infusion fluid for a first period of time and cause the fluid pressure source to apply a negative pressure to the first fluid storage container for a second period of time following the first period of time.

It is to be understood that both the foregoing general description and the following detailed description are exemplary and explanatory in nature and are intended to provide an understanding of the present disclosure without limiting the scope of the present disclosure. In that regard, additional aspects, features, and advantages of the present disclosure will be apparent to one skilled in the art from the following detailed description.

### BRIEF DESCRIPTION OF THE DRAWINGS

The accompanying drawings illustrate the devices and methods disclosed herein and together with the description, serve to explain the principles of the present disclosure.
Fig. 1 is a diagram showing an illustrative ophthalmic surgical system that includes a fluid delivery system.
Fig. 2 is a diagram showing an illustrative fluid delivery system with a fluid storage container.
Fig. 3 is a diagram showing an embodiment of a fluid delivery system with dual fluid storage containers according to the invention.
Fig. 4 is a flowchart showing a process for providing fluid through the fluid delivery system with two fluid storage containers.
Fig. 5A is a diagram showing an illustrative fluid delivery bag capable of being degassed before infusion.
Fig. 5B is a diagram showing another illustrative fluid delivery bag capable of being degassed before infusion.
Fig. 6 is a flowchart showing an illustrative method for degassing fluid before infusion into a patient's eye.

### DETAILED DESCRIPTION

For the purposes of promoting an understanding of the principles of the present disclosure, reference will now be made to the embodiments illustrated in the drawings, and specific language will be used to describe the same. It will nevertheless be understood that no limitation of the scope of the disclosure is intended. Any alterations and further modifications to the described devices, instruments, methods, and any further application of the principles of the present disclosure are fully contemplated as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For simplicity, in some instances the same reference numbers are used throughout the drawings to refer to the same or like parts.

The present disclosure is directed to a fluid delivery system and a fluid storage container adapted to degas a fluid within a fluid storage container before that fluid is infused into the patient's eye. According to some examples, the fluid storage container includes an infusion outflow port and a pressure inlet. The pressure inlet includes a filter such as a semi-permeable membrane. Before the fluid is infused into the patient's eye, a vacuum is applied through the filter. The filter prevents the liquid infusion fluid from exiting the bag, while permitting passage of gas bubbles through the filter. In this manner, the infusion fluid can be degassed before it is infused into the patient's eye. The fluid delivery system and fluid storage container will be described in further detail below.

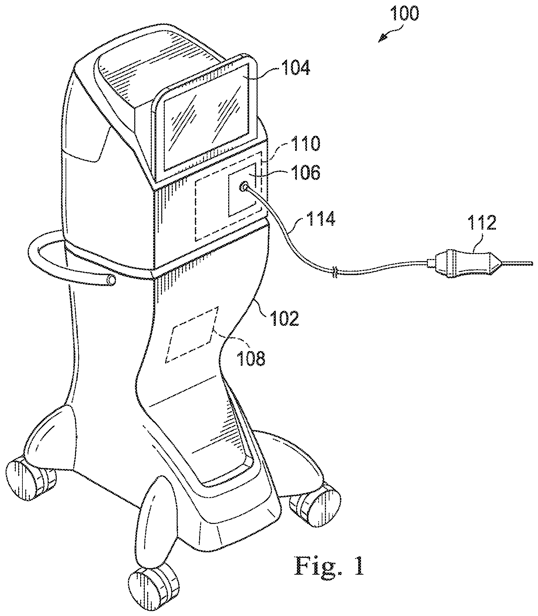

Fig. 1 is a diagram showing an illustrative ophthalmic surgical system 100. According to the present example, the ophthalmic surgical system 100 includes a surgical console 102 and an ophthalmic surgical tool 112. The ophthalmic surgical tool 112 is in fluid communication with the console 102 through a fluid infusion line 114. The surgical console 102 includes a display screen 104 and a fluid delivery system 110. In one implementation, the surgical console 102 is configured to be mobile and may be used by a user, such as a health care provider, to perform ophthalmic surgical procedures. The surgical console 102 may also include a control system 108 that may be configured to process, receive, and store data to perform various functions associated with the ophthalmic surgical tool 112.

The display screen 104 may communicate information to the user, and in some implementations, may show data relating to system operation and performance during a surgical procedure. In some examples, the display screen 104 is a touchscreen that allows the operator to interact with the surgical console 102 through a graphical user interface.

The fluid delivery system 110 may include a cassette 106 that is removably insertable into the fluid delivery system 100. In some examples, the cassette 106 may include components of the fluid delivery system 110 that may come into contact with patient fluids and tissue. Specifically, the cassette 106 may include a fluid storage container and the fluid infusion line 114. In some examples, the cassette 106 may include components of other systems such as an aspiration system (not shown).

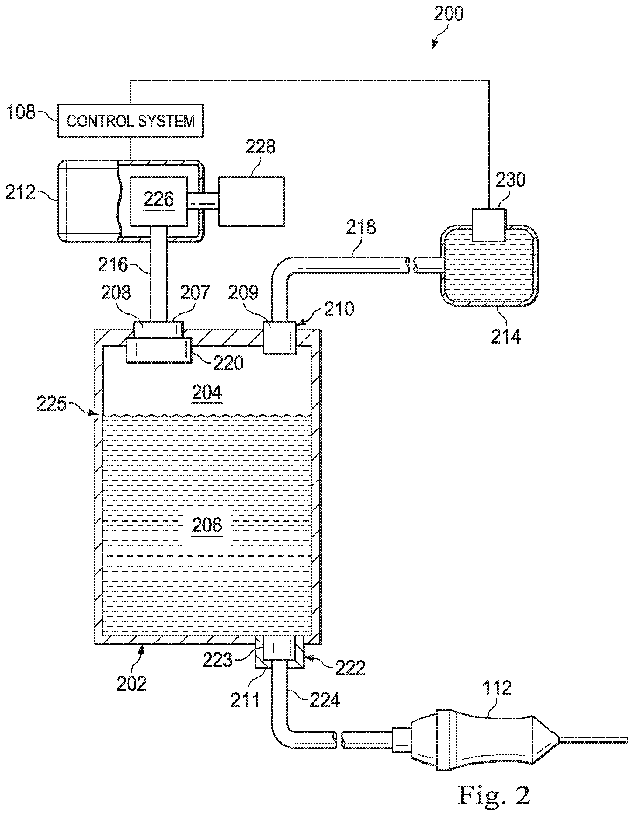

Fig. 2 is a diagram showing an illustrative fluid delivery system 200 with a fluid storage container 202. The fluid delivery system 200 may correspond to the fluid delivery system 110 described above. According to the present example, the fluid delivery system 200 includes a pressure source 212 having a pump 226, a fluid source 214, and a fluid infusion line 224. The fluid storage container 202 includes a fluid chamber 204 for storing infusion fluid 206, a pressure inlet 208 with a filter 220, a fluid inflow port 210, and a fluid outflow port 222.

As described above, the fluid delivery system 200 may utilize a cassette (e.g. 106, Fig. 1) that is insertable into the surgical console (e.g. 102, Fig. 1). The cassette may include the fluid storage container 202 and the fluid infusion line 224. The cassette may be structurally configured such that, when inserted into the console, the fluid inflow port 210 and pressure inlet 208 are appropriately connected to the fluid source 214 and the pressure source 212, respectively.

The pressure source 212 may be a compressor or pump that is integrated into the surgical console (e.g., 102, Fig. 1). In some examples, the pressure source 212 may be provided by a machine (e.g., a pump) separate from the console that is connectable to the surgical console through a pressure line. In either case, the pressure source 212 is connectable to the pressure inlet 208 of the fluid storage container 202 through the pressure line 216, such as a conduit. The pressure line 216 provides fluid communication between the pressure inlet 208 and the pressure source 212.

The pressure source 212 is configured to apply both positive and negative pressure relative to atmospheric pressure to the fluid storage container 202 through the pressure inlet 208. Specifically, the pressure source 212 is adapted to apply a negative pressure (i.e., a vacuum) to the fluid storage container 202 to degas the infusion fluid 206 stored therein. The pressure source 212 is also adapted to apply positive pressure to push the infusion fluid 206 out of the fluid storage container 202, through the fluid infusion line 224 and into a patient's eye.

In some examples, including the exemplary implementation in Fig. 2, the pressure source 212 utilizes a Venturi pump 226 to apply negative pressure to the fluid storage container. In some examples, to apply a vacuum to the pressure inlet 208, the pressure source causes a fluid to flow from the pressure source 212, through the Venturi pump 226, and to a muffler 228. The Venturi pump 226 includes a narrower portion of tubing. Because speed increases through the narrow portion, pressure drops, thereby creating a vacuum. In this manner, the pressure source 212 can apply negative pressure to the pressure inlet 208. Other types of pumps for providing a vacuum may be used as well.

While the exemplary implementation in Fig. 2 uses a single interface between the container 202 and the pressure source 212, other implementations include two separate pressure interfaces; one for having positive pressure applied and one for having negative pressure applied. In such a case, two separate pressure lines may connect the two pressure interfaces to the pressure source 212. In some cases, the pressure source that provides a positive pressure may be a separate piece of machinery than the pressure source that provides a negative pressure. In other words, there may be two separate pressure sources; one for providing positive pressure and one for providing negative pressure. For example, a pump may provide vacuum or negative pressure and a compressor may provide positive pressure.

The fluid source 214 provides an infusion fluid to the fluid storage container 202. The infusion fluid may be, for example, a balanced salt solution (BSS). The infusion fluid may be provided to the fluid storage container 202 in any of a variety of manners. In one example, the fluid source 214 may include a fluid tank that is sized to hold a substantially larger quantity of fluid than the fluid storage container 202. Such a fluid tank may then be used to fill the fluid storage container 202 with the infusion fluid as needed. In some examples, the fluid source 214 may be a fluid tank that is external to the surgical console 102. In either case, the fluid storage container 202 is connectable to the fluid source 214 through a fluid line 218. The fluid line 218 thus provides fluid communication between the fluid source 214 and the fluid inflow interface 210 of the fluid storage container 202.

Still referring to Fig. 2, the chamber 204 of the fluid storage container 202 is filled with an infusion fluid 206. In some examples, the chamber 204 may be sized such that the amount of fluid within the chamber 206 is generally sufficient for a single surgical procedure. In some examples, however, the chamber 204 may be sized to hold a smaller quantity of infusion fluid. In such a case, the chamber 204 may be refilled during a surgical procedure.

The pressure inlet 208 may include a pressure interface 207 that allows the pressure inlet 208 to connect with the pressure line 216 such that a fluid-tight seal is formed. The pressure interface 207 may be a selectively attachable interface, such as a quick disconnect fitting or other interface. In some examples, the pressure interface 207 is a Luer fitting. The pressure inlet 208 allows fluid communication between the chamber 204 and the pressure source 212. According to the present example, the pressure inlet 208 includes the filter 220. The filter 220 is structurally configured to allow gaseous fluid to pass therethrough and prevent liquid fluid from passing therethrough. Thus, when a vacuum, such as negative pressure, is applied to the pressure inlet 208, the gas within the chamber 204 and gas bubbles within the infusion fluid 206 can be pulled through the filter 220. But, the infusion fluid 206 (i.e., BSS) is maintained within the fluid storage container and not pulled through the filter 220.

The fluid inflow port 210 includes an interface 209 that allows the fluid inflow port 210 to connect to the fluid line 218 such that a fluid-tight seal is formed. The interface 209 may be a selectively attachable interface, such as a quick disconnect fitting or other interface. In some examples, the interface 209 is a Luer fitting. The fluid line 218 provides fluid communication between the fluid source 214 and the fluid inflow port 210. The fluid inflow port 210 allows fluid communication between the chamber 204 and the fluid line 218. In some examples, the fluid inflow port 210 may include a one-way valve that allows fluid to flow into the chamber 204 but prevents fluid from flowing out of the chamber 204.

The fluid outflow port 222 includes an interface 211 that allows the fluid outflow port 222 to connect to the fluid infusion line 224 such that a fluid-tight seal is formed. The fluid outflow port 222 thus provides fluid communication between the chamber 204 and the fluid infusion line 224. The fluid infusion line 224 provides fluid communication between the fluid outflow port 222 and the ophthalmic surgical tool 112 that injects the infusion fluid into the patient's eye. In some examples, the fluid outflow port 222 may include a one-way valve that allows fluid to flow out of the chamber 204 but prevents fluid from flowing into the chamber 204. The fluid outflow port 222 may also include a stop valve 223 or check valve to selectively prevent or allow fluid from flowing through the fluid outflow port 222.

During operation of the fluid delivery system, the control system (e.g. 108, Fig. 1) may manage the various components to direct fluid as desired. For example, after the cassette is inserted into the surgical console 102, the chamber 204 may be empty. Thus, the control system 108 may operate a pump 230 to cause the fluid from the fluid source 214 to be pumped into the chamber 204 to fill the chamber. During this time, the stop valve 223 of the fluid outflow port 222 may be closed so as to prevent fluid from flowing out of the fluid outflow port 222. In some examples, the infusion fluid 206 may be pumped into the chamber 204 until the fluid level reaches a certain threshold level 225 that is lower than the top of the chamber 204.

After the chamber 204 has been appropriately filled with infusion fluid 206, the control system 108 may apply a negative pressure to the pressure inlet 208 through use of the pressure source 212 and the pump 226. The negative pressure, or vacuum, that is applied can degas the infusion fluid 206 stored within the chamber 204. In other words, gas bubbles within the infusion fluid solution may be removed from the infusion fluid solution.

After the infusion fluid 206 has been degassed, the solution may be ready for infusion into the patient's eye. The control system 108 may thus apply positive pressure to the pressure inlet 208. During this time, the stop valve 223 of the fluid outflow port 222 may be open so as to allow fluid flow therethrough. The positive pressure at the pressure inlet 208 causes the infusion fluid to be pushed out of the chamber 204, into the fluid infusion line 224, through the ophthalmic surgical tool 112, and into the patient's eye. The magnitude of the positive pressure may be controlled so as to provide the desired flow rate of infusion fluid 206 to the patient's eye.

The control system 108 may include a processor and a memory. The memory may store machine readable instructions that when executed by the processor, cause the control system 108 to perform various tasks. For example, the control system 108 may send control signals to the pressure source 212 and the fluid source 214. Such control signals may activate either the pressure source 212 or the fluid source 214 to behave as desired at designated points in time. For example, the control system 108 may cause the fluid source 214 to fill the fluid storage container 202 with fluid 206. Then, the control system 108 may cause the pressure source 212 to apply a negative pressure to degas the fluid 206 within the fluid storage container 202. The control system 108 may then cause the pressure source 212 to apply positive pressure to the fluid storage container 202 to push the fluid 206 out of the fluid storage container 202.

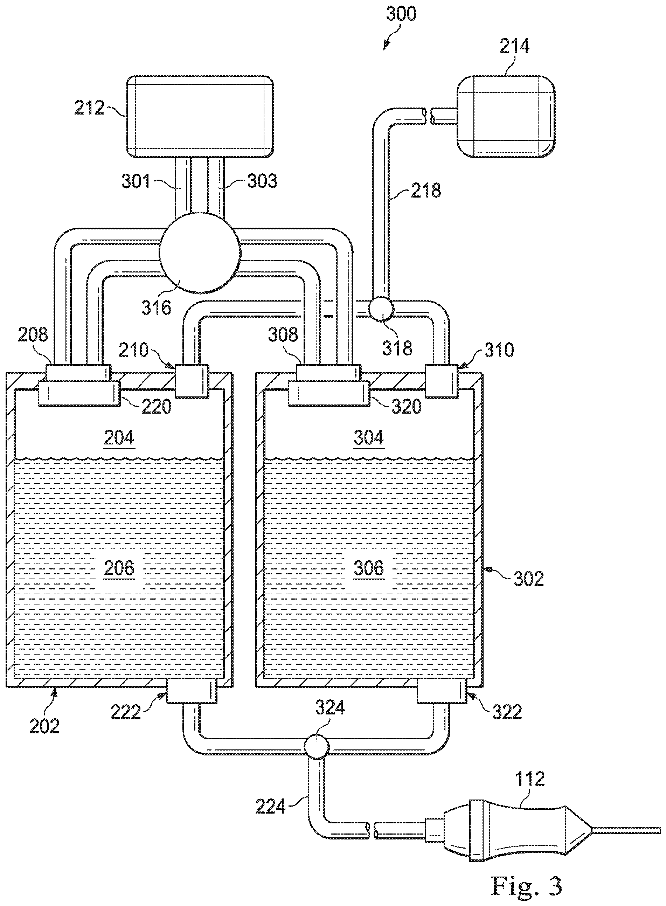

Fig. 3 is a diagram showing an embodiment of a fluid delivery system 300 with dual fluid storage containers 202, 302. Like the first fluid storage container 202, the second fluid storage container 302 includes a chamber 304 adapted to hold a quantity of fluid 306, a pressure inlet 308 with a filter 320, a fluid inflow port 310, and a fluid outflow port 322. In this example, however, the fluid line 218 includes a switch valve 318 that allows fluid from the fluid source 214 to be directed to either the first fluid storage container 202 or the second fluid storage container 302. Similarly, the fluid infusion line 224 includes a switch valve 324 that allows fluid from either the first fluid storage container 202 or the second fluid storage container 302 to be directed to the ophthalmic surgical tool 112.

According to the present example, the pressure lines 301, 303 connect the pressure source to the pressure inlets 208, 308 of the fluid storage containers 202, 302 through a switch valve 316. The first pressure line 301 may be used for applying positive pressure and the second pressure line 303 may be used for applying negative pressure. Thus, positive pressure may be applied to one fluid storage container while negative pressure is applied to the other and vice versa.

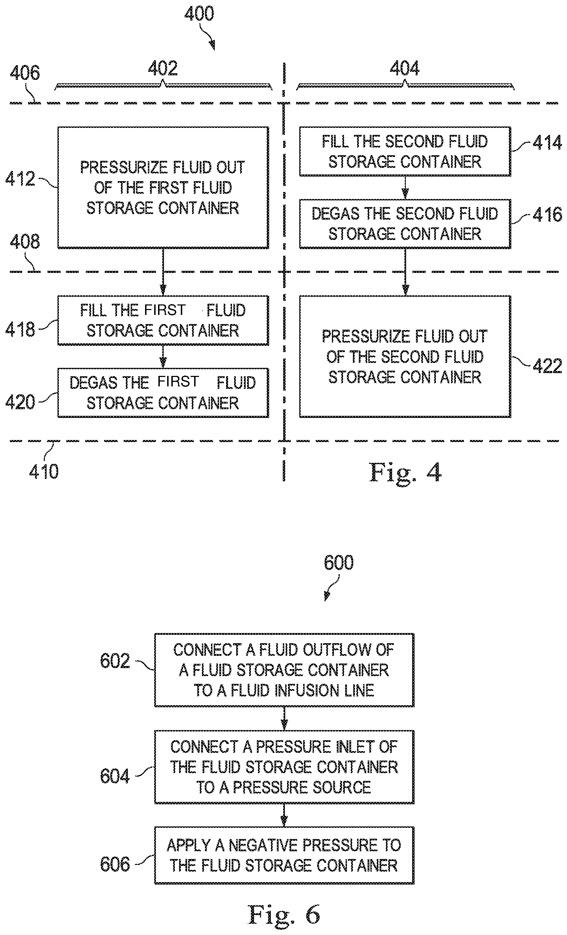

Fig. 4 is a flowchart showing a process 400 for providing fluid through the fluid delivery system 300 that includes the two fluid storage containers 202, 302. Steps performed on the first fluid storage container 202 are shown in the left column 402 and the steps performed on the second fluid storage container 302 are shown in the right column 404. Reference numeral 406 identifies a starting point in time, where the first fluid storage container 202 is filled with infusion fluid and the second fluid storage container 302 is empty.

Between points in time identified by the reference numerals 406 and 408, at step 412, infusion fluid is pushed out of the first fluid storage container 202 by applying positive pressure to the pressure inlet 208 of the first fluid storage container 202. Meanwhile, steps 414 and 416 are performed on the second fluid storage container 302. At step 414, the second fluid storage container 302 is filled with infusion fluid. After the second fluid storage container 302 is appropriately filled, at step 416, the infusion fluid solution within the second fluid storage container 302 is degassed by applying a negative pressure to the pressure inlet 308 of the second fluid storage container 302.

At the point in time 408, after the fluid in the first fluid storage container 202 falls below a minimum fluid level, the process 400 switches. Specifically, between time points 408 and 410, at step 422, infusion fluid is pushed out of the second fluid storage container 302 by applying positive pressure to the pressure inlet 308. Meanwhile, steps 418 and 420 are performed on the first fluid storage container 202. At step 418, the first fluid storage container 202 is filled with infusion fluid. After the first fluid storage container 202 is appropriately filled, at step 420, the infusion fluid solution within the first fluid storage container 202 is degassed by applying a negative pressure to the pressure inlet 208 of the first fluid storage container 202.

At time point 410, the first fluid storage container 202 is filled with degassed fluid and the second fluid storage container 302 is empty. The process 400 may continue by switching between the fluid storage containers 202, 302 as described above. Specifically, while infusion fluid is being pressurized out of one fluid storage container, the other fluid storage container is being filled and degassed. Thus, a steady flow of degassed infusion fluid can be provided to the patient's eye.

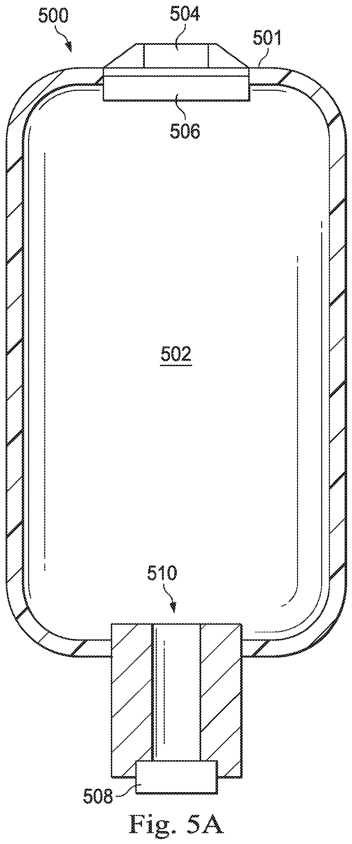

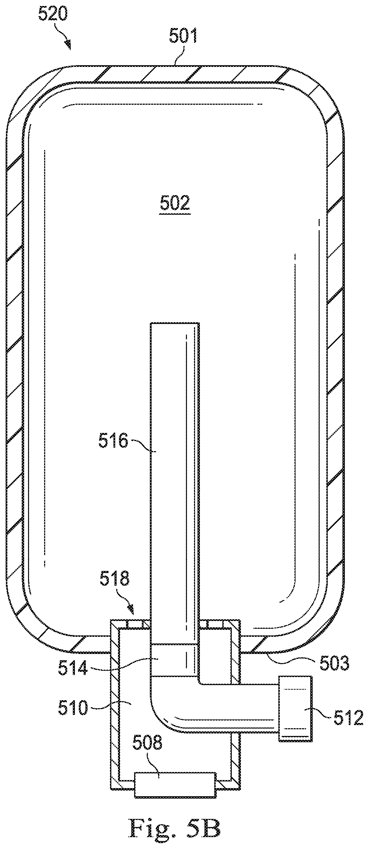

Figs. 5A and 5B are diagrams showing illustrative fluid delivery bags capable of being degassed before infusion. The fluid delivery bags may store an infusion fluid. The bags may be flexible so that when squeezed, the infusion fluid within is pressed out of the bag and into the patient's eye. For example, some surgical consoles (e.g., 102, Fig. 1) may include an actuator mechanism that squeezes a bag filled with fluid in order to provide pressurized infusion fluid into the patient's eye.

Fig. 5A illustrates an example in which the degassing interface 504 is at a top 501 of a bag 500. In the present example, the bag 500 includes the degassing interface 504 with a filter 506, an interior chamber 502, and a fluid outflow port 510 with an outflow interface 508. The outflow interface 508 may be connectable to a fluid infusion line (e.g., 224, Fig. 2). The degassing interface 504 may be connectable to a pressure source (e.g. 212, Fig. 2) through a pressure line. In this example, the degassing interface 504 includes a filter 506 that may allow gaseous fluids to pass through preventing liquid fluids from passing through. The filter 506 may be, for example, a semipermeable membrane. Thus, when a negative pressure is applied to the degassing interface 504, gas bubbles within the infusion fluid solution may be pulled out of the solution. But, the infusion fluid does not pass through the filter 506. Thus, the infusion fluid solution within the chamber 502 can be degassed.

Fig. 5B illustrates an example in which the degassing interface 512 is at a bottom 503 of a bag 520. In the present example, the bag 520 includes the degassing interface 512 with a filter 514, and a fluid outflow port 510 with an outflow interface 508. The bag also includes a snorkel 516 that extends into the center portion of the chamber 502. The fluid outflow port 510 may include an annular channel 518 that surrounds the snorkel 516. The outflow interface 508 may be connectable to a fluid infusion line (e.g., 224, Fig. 2). Thus, fluid flowing out of the chamber 502 passes through the annular channel 518, through the outflow interface 508, and into a fluid infusion line. The degassing interface 512 may be connectable to a pressure source (e.g. 212, Fig. 2) through a pressure line. The degassing interface 504 includes a filter 514 that may allow gaseous fluids to pass through preventing liquid fluids from passing through. The filter 514 may be, for example, a semipermeable membrane. Thus, when a negative pressure is applied to the degassing interface 504, gas bubbles within the infusion fluid solution may be pulled out the solution. Specifically, infusion fluid within the chamber 502 is pulled through the snorkel 516 against the filter 514. But, the infusion fluid is not pulled through the degassing filter 514. Thus, the infusion fluid solution within the chamber 502 can be degassed.

The snorkel 516 may be any suitable length. For example, the snorkel 516 may be relatively short and extend only a small distance from the bottom 503 of the bag 520. In some examples, the snorkel 516 may be relatively long and extend to a point near the top 501 of the bag 520. In some examples, both degassing interface 504 as illustrated in Fig. 5A and degassing interface 512 as illustrated in Fig. 5B may be included within a single bag.

Fig. 6 is a flowchart illustrating a method 600 for degassing fluid before infusion into a patient's eye. According to the present example, the method includes a step 602 for connecting a fluid outflow port of a fluid storage container to a fluid infusion line. The fluid infusion line provides fluid communication between the fluid storage container and an ophthalmic surgical tool.

At a step 604, the pressure inlet of the fluid storage container is connected to a pressure source. The pressure source is capable of applying both positive and negative pressure to the fluid storage container. Additionally, the pressure inlet includes a filter, such as a semipermeable membrane, that allows gas to pass therethrough but prevents liquid from flowing therethrough.

At a step 606, the pressure source applies negative pressure to the pressure inlet. By applying negative pressure to the pressure inlet, gas within the chamber of the fluid storage container will exit the chamber through the membrane. Additionally, gas bubbles within the infusion fluid solution will exit the chamber. In other words, the infusion fluid solution is degassed. Through use of principles described herein, infusion fluid to be degassed before this injected into the patient's eye. This can help improve the visibility for the operator during a surgical procedure.

Persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. A fluid delivery system (200) for providing pressurized fluid for infusion into a patient's eye, comprising:
a pressure source (212) capable of selectively producing positive and negative fluid pressure in a first fluid storage container and a second fluid storage container;
an infusion fluid source (214);
a fluid infusion line (224);
a first fluid storage container (202) comprising:
a first chamber (204);
a first fluid outflow port (222) in communication with the fluid infusion line to provide fluid communication between the first chamber and the fluid infusion line;
a first pressure inlet (208) in communication with the pressure source (212); and
a first filter (220) disposed between the first pressure inlet and the first chamber; and
a second fluid storage container (302) comprising:
a second chamber (304);
a second fluid outflow port (322) in communication with the fluid infusion line to provide fluid communication between the second chamber and the fluid infusion line;
a second pressure inlet (308) in communication with the pressure source (212); and
a second filter (320) disposed between the second pressure inlet (308) and the second chamber (304);
wherein the infusion fluid source (214) is in communication with the first and second fluid storage containers;
and
a control system (108) configured to fill the first fluid storage container (202) and the second fluid storage container (302) with infusion fluid from the infusion fluid source (214); and
the control system (108) configured to cause the pressure source to selectively apply negative pressure or positive pressure to the first chamber (204) and the second chamber (304) such that the negative pressure acts on the infusion fluid in one of the first and second chamber to degas the infusion fluid and the positive pressure acts to push the infusion fluid out of the other of the first or second chamber and into the fluid infusion line (224);
wherein the control system (108) is configured to apply positive pressure to the first fluid storage container (202) during a period of time, and during the period of time, sequentially cause the second fluid storage container (302) to be filled with infusion fluid and apply a negative pressure to the second fluid storage container (302).

2. The fluid delivery system of claim 1, wherein the first fluid storage container (202) and the second fluid storage container (302) further comprise fluid inflow ports (210) connectable to the fluid source.

3. The fluid delivery system of claim 1, wherein the first and second filters (320) comprises a semipermeable membrane.

4. A method of operating the system (200) for providing pressurized fluid for infusion into a patient's eye of claim 1: the method comprising:
filling (406) the first fluid storage container (202) with infusion fluid from the infusion fluid source (214);
applying a negative pressure to the first fluid storage container (202) to degas the infusion fluid within the first fluid storage container;
with the pressure source (212), applying (412) a positive pressure to the first fluid storage container (202) to push the infusion fluid out of the first fluid storage container through the first fluid outflow port (222) and into the fluid infusion line (224);
while applying a positive pressure to the first fluid storage container (202), filling (414) a second fluid storage container (302) with infusion fluid and applying (416) a negative pressure to the second fluid storage container to degas the infusion fluid in the second fluid storage container (302);
discontinuing applying the positive pressure to the first fluid storage container (202);
with the pressure source (212), applying (422) a positive pressure to the second fluid storage container (202) to push fluid out of the second fluid storage container (302) through the second fluid outflow port (322) and into the fluid infusion line (224).

5. The method of claim 4, further comprising:
connecting a fluid inflow port (210) of the first fluid storage container to a fluid source of the fluid delivery system; and
before applying the negative pressure, filling the first fluid storage container with infusion fluid from the fluid source.

6. The method of claim 4, further comprising, pushing (422) fluid out of a second fluid storage container (302) while both filling (418) the first fluid storage container (202) and degassing (420) the fluid in the first fluid storage container.

## Patentansprüche

1. Fluidzufuhrsystem (200) zur Bereitstellung von unter Druck stehendem Fluid zur Infusion in das Auge eines Patienten, umfassend:
eine Druckquelle (212), die gezielt Fluid-Überdruck und -Unterdruck in einem ersten Fluidvorratsbehälter und einem zweiten Fluidvorratsbehälter erzeugen kann,
eine Infusionsfluidquelle (214),
eine Fluidinfusionsleitung (224),
einen ersten Fluidvorratsbehälter (202), der Folgendes umfasst:
eine erste Kammer (204),
eine erste Fluidabströmöffnung (222) in Kommunikation mit der Fluidinfusionsleitung, um eine Fluidverbindung zwischen der ersten Kammer und der Fluidinfusionsleitung bereitzustellen,
einen ersten Druckeinlass (208) in Kommunikation mit der Druckquelle (212) und
einen ersten Filter (220), der zwischen dem ersten Druckeinlass und der ersten Kammer angeordnet ist,
und
einen zweiten Fluidvorratsbehälter (302), der Folgendes umfasst:
eine zweite Kammer (304),
eine zweite Fluidabströmöffnung (322) in Kommunikation mit der Fluidinfusionsleitung, um eine Fluidverbindung zwischen der zweiten Kammer und der Fluidinfusionsleitung bereitzustellen,
einen zweiten Druckeinlass (308) in Kommunikation mit der Druckquelle (212) und
einen zweiten Filter (320), der zwischen dem zweiten Druckeinlass (308) und der zweiten Kammer (304) angeordnet ist,
wobei die Infusionsfluidquelle (214) mit dem ersten und dem zweiten Fluidvorratsbehälter kommuniziert,
und
ein Steuersystem (108), das dazu ausgestaltet ist, den ersten Fluidvorratsbehälter (202) und den zweiten Fluidvorratsbehälter (302) mit Infusionsfluid von der Infusionsfluidquelle (214) zu füllen, und
wobei das Steuersystem (108) dazu ausgestaltet ist zu veranlassen, dass die Druckquelle die erste Kammer (204) und die zweite Kammer (304) gezielt mit Unterdruck oder Überdruck zu beaufschlagen, so dass der Unterdruck auf das Infusionsfluid in der ersten oder der zweiten Kammer einwirkt, um das Infusionsfluid zu entgasen, und der Überdruck dahingehend wirkt, das Infusionsfluid aus der jeweils anderen der ersten und der zweiten Kammer und in die Fluidinfusionsleitung (224) zu drücken,
wobei das Steuersystem (108) dazu ausgestaltet ist, den ersten Fluidvorratsbehälter (202) während eines Zeitraums mit Überdruck zu beaufschlagen und während des Zeitraums nacheinander zu veranlassen, dass der zweite Fluidvorratsbehälter (302) mit Infusionsfluid gefüllt wird, und den zweiten Fluidvorratsbehälter (302) mit einem Unterdruck zu beaufschlagen.

2. Fluidzufuhrsystem nach Anspruch 1, wobei der erste Fluidvorratsbehälter (202) und der zweite Fluidvorratsbehälter (302) ferner Fluideinströmöffnungen (210) umfassen, die mit der Fluidquelle verbindbar sind.

3. Fluidzufuhrsystem nach Anspruch 1, wobei der erste und der zweite Filter (320) ein semipermeable Membran umfassen.

4. Verfahren zum Betreiben des Systems (200) zur Bereitstellung von unter Druck stehendem Fluid zur Infusion in das Auge eines Patienten nach Anspruch 1:
wobei das Verfahren Folgendes umfasst:
Füllen (406) des ersten Fluidvorratsbehälters (202) mit Infusionsfluid von der Infusionsfluidquelle (214),
Beaufschlagen des ersten Fluidvorratsbehälters (202) mit einem Unterdruck, um das Infusionsfluid in dem ersten Fluidvorratsbehälter zu entgasen,
mit der Druckquelle (212) Beaufschlagen (412) des ersten Fluidvorratsbehälters (202) mit einem Überdruck, um das Infusionsfluid aus dem ersten Fluidvorratsbehälter durch die erste Fluidabströmöffnung (222) und in die Fluidinfusionsleitung (224) zu drücken,
während des Beaufschlagens des ersten Fluidvorratsbehälters (202) mit einem Überdruck Füllen (414) eines zweiten Fluidvorratsbehälters (302) mit Infusionsfluid und Beaufschlagen (416) des zweiten Fluidvorratsbehälters mit einem Unterdruck, um das Infusionsfluid in dem zweiten Fluidvorratsbehälter (302) zu entgasen,
Einstellen des Beaufschlagens des ersten Fluidvorratsbehälters (202) mit dem Überdruck,
mit der Druckquelle (212) Beaufschlagen (422) des zweiten Fluidvorratsbehälters (202) mit einem Überdruck, um Fluid aus dem zweiten Fluidvorratsbehälter (302) durch die zweite Fluidabströmöffnung (322) und in die Fluidinfusionsleitung (224) zu drücken.

5. Verfahren nach Anspruch 4, ferner umfassend:
Verbinden einer Fluideinströmöffnung (210) des ersten Fluidvorratsbehälters mit einer Fluidquelle des Fluidzufuhrsystems und
vor dem Beaufschlagen mit dem Unterdruck Füllen des ersten Fluidvorratsbehälters mit Infusionsfluid von der Fluidquelle.

6. Verfahren nach Anspruch 4, ferner umfassend Drücken (422) von Fluid aus einem zweiten Fluidvorratsbehälter (302), während sowohl der erste Fluidvorratsbehälter (202) gefüllt und das Fluid in dem ersten Fluidvorratsbehälter entgast wird.

## Revendications

1. Système de distribution de fluide (200) pour fournir un fluide sous pression destiné à être perfusé dans l'œil d'un patient, comprenant :
une source de pression (212) capable de produire sélectivement une pression de fluide positive et négative dans un premier récipient de stockage de fluide et un second récipient de stockage de fluide ;
une source de fluide de perfusion (214) ;
une ligne de perfusion de fluide (224) ;
un premier récipient de stockage de fluide (202) comprenant :
une première chambre (204) ;
un premier orifice de sortie de fluide (222) en communication avec la ligne de perfusion de fluide pour fournir une communication fluidique entre la première chambre et la ligne de perfusion de fluide ;
une première entrée de pression (208) en communication avec la source de pression (212) ; et
un premier filtre (220) disposé entre la première entrée de pression et la première chambre ; et
un second récipient de stockage de fluide (302) comprenant :
une seconde chambre (304) ;
un second orifice de sortie de fluide (322) en communication avec la ligne de perfusion de fluide pour fournir une communication fluidique entre la seconde chambre et la ligne de perfusion de fluide ;
une seconde entrée de pression (308) en communication avec la source de pression (212) ; et
un second filtre (320) disposé entre la seconde entrée de pression (308) et la seconde chambre (304) ;
la source de fluide de perfusion (214) étant en communication avec les premier et second récipients de stockage de fluide ; et
un système de commande (108) configuré pour remplir le premier récipient de stockage de fluide (202) et le second récipient de stockage de fluide (302) avec du fluide de perfusion provenant de la source de fluide de perfusion (214) ; et
le système de commande (108) étant configuré pour amener la source de pression à appliquer sélectivement une pression négative ou une pression positive à la première chambre (204) et à la seconde chambre (304) de telle sorte que la pression négative agisse sur le fluide de perfusion dans l'une des première et seconde chambres pour dégazer le fluide de perfusion et que la pression positive agisse pour pousser le fluide de perfusion hors de l'autre des première et seconde chambres et dans la ligne de perfusion de fluide (224) ;
le système de commande (108) étant configuré pour appliquer une pression positive au premier récipient de stockage de fluide (202) pendant une période de temps et, pendant la période de temps, provoquer séquentiellement le remplissage du second récipient de stockage de fluide (302) avec du fluide de perfusion et appliquer une pression négative au second récipient de stockage de fluide (302).

2. Système de distribution de fluide selon la revendication 1, le premier récipient de stockage de fluide (202) et le second récipient de stockage de fluide (302) comprenant en outre des orifices d'entrée de fluide (210) pouvant être reliés à la source de fluide.

3. Système de distribution de fluide selon la revendication 1, les premier et second filtres (320) comprenant une membrane semi-perméable.

4. Procédé de fonctionnement du système (200) pour fournir un fluide sous pression destiné à être perfusé dans l'œil d'un patient selon la revendication 1,
le procédé comprenant :
le remplissage (406) du premier récipient de stockage de fluide (202) avec du fluide de perfusion provenant de la source de fluide de perfusion (214) ;
l'application d'une pression négative au premier récipient de stockage de fluide (202) pour dégazer le fluide de perfusion à l'intérieur du premier récipient de stockage de fluide ;
avec la source de pression (212), l'application (412) d'une pression positive au premier récipient de stockage de fluide (202) pour pousser le fluide de perfusion hors du premier récipient de stockage de fluide à travers le premier orifice de sortie de fluide (222) et dans la ligne de perfusion de fluide (224) ;
pendant l'application d'une pression positive au premier récipient de stockage de fluide (202), le remplissage (414) d'un second récipient de stockage de fluide (302) avec du fluide de perfusion et l'application (416) d'une pression négative au second récipient de stockage de fluide pour dégazer le fluide de perfusion dans le second récipient de stockage de fluide (302) ;
l'arrêt de l'application de la pression positive au premier récipient de stockage de fluide (202) ;
avec la source de pression (212), l'application (422) d'une pression positive au second récipient de stockage de fluide (202) pour pousser le fluide hors du second récipient de stockage de fluide (302) à travers le second orifice de sortie de fluide (322) et dans la ligne de perfusion de fluide (224).

5. Procédé selon la revendication 4, comprenant en outre :
la liaison d'un orifice d'entrée de fluide (210) du premier récipient de stockage de fluide à une source de fluide du système de distribution de fluide ; et
avant l'application de la pression négative, le remplissage du premier récipient de stockage de fluide avec du fluide de perfusion provenant de la source de fluide.

6. Procédé selon la revendication 4, comprenant en outre l'action de pousser (422) le fluide hors d'un second récipient de stockage de fluide (302) tout en remplissant (418) le premier récipient de stockage de fluide (202) et en dégazant (420) le fluide dans le premier récipient de stockage de fluide.
